(19) Europäisches Patentamt
European Patent Office
Office européen des brevets

(11) EP 1 629 032 B1

(12) # EUROPÄISCHE PATENTSCHRIFT

(45) Veröffentlichungstag und Bekanntmachung des Hinweises auf die Patenterteilung:
**27.02.2019 Patentblatt 2019/09**

(21) Anmeldenummer: **04741624.3**

(22) Anmeldetag: **21.05.2004**

(51) Int Cl.:
***A61L 15/28*** *(2006.01)* ***C08L 5/04*** *(2006.01)*
***A61K 8/365*** *(2006.01)*

(86) Internationale Anmeldenummer:
**PCT/EP2004/050883**

(87) Internationale Veröffentlichungsnummer:
**WO 2004/104076 (02.12.2004 Gazette 2004/49)**

(54) **VERFAHREN ZUR HERSTELLUNG VON ALGINAT-HALTIGEN PORÖSEN FORMKÖRPERN**

METHOD FOR PRODUCING POROUS MOULDED BODIES CONTAINING ALGINATE

PROCEDE DE PRODUCTION DE CORPS MOULES POREUX CONTENANT DE L'ALGINATE

(84) Benannte Vertragsstaaten:
**AT BE BG CH CY CZ DE DK EE ES FI FR GB GR HU IE IT LI LU MC NL PL PT RO SE SI SK TR**

(30) Priorität: **23.05.2003 DE 10323794**

(43) Veröffentlichungstag der Anmeldung:
**01.03.2006 Patentblatt 2006/09**

(73) Patentinhaber: **MedSkin Solutions Dr. Suwelack AG**
**48727 Billerbeck (DE)**

(72) Erfinder: **MALESSA, Ralf**
**45134 Essen (DE)**

(74) Vertreter: **CH Kilger Anwaltspartnerschaft mbB**
**Fasanenstrasse 29**
**10719 Berlin (DE)**

(56) Entgegenhaltungen:
**DE-B- 1 203 590** **DE-U- 20 219 660**
**GB-A- 560 317** **SU-A- 658 148**
**US-A- 3 060 032** **US-A- 5 211 980**
**US-A- 5 718 916**

EP 1 629 032 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

STAND DER TECHNIK

Die Erfindung betrifft Verfahren zur Herstellung von Alginat- haltigen porösen bzw. schwammartigen Formkörpern.

Es ist bekannt, dass Alkalialginate, wie z. B. Na-Alginat, wasserlöslich, Erdalkali-Alginate, wie z. B. Ca-Alginat, hingegen wasserunlöslich sind. Dünne wasserunlösliche Schichten lassen sich daher beispielsweise durch Besprühen eines dünnen Na-Alginatfilmes mit einer $CaCl_2$-Lösung herstellen.

Will man hingegen dickerer Schichten herstellen, so besteht die Schwierigkeit darin, dass die homogene Einarbeitung freier Ca-Ionen in eine Na-Alginatlösung durch eine starke Zunahme der Lösungsviskosität erschwert wird, so dass keine gleichmäßigen Produkte, sondern unzusammenhängende Ca-Alginat- Agglomerate entstehen.

Um dieses Problem zu überkommen schlägt die US 5718916 beispielsweise vor, die wässrige Lösung der wasserlöslichen Alginat- Zusammensetzung mit einem wasserlöslichen Komplexierungsmittel, wie z. B. Natriumzitrat zu versetzen. Wird anschließend beispielsweise ein leicht lösliches Calciumsalz wie Calciumchlorid hinzugegeben, so wird durch die Anwesenheit des Komplexierungsmittels die sofortige Ausfällung von Calciumalginat verzögert, wodurch die Bildung von unlöslichen Calciumalginat-Kügelchen in dem Produkt verhindert werden soll. In den Beispielen der genannten US-Patentschrift wird jedoch in Maßstäben von wenigen Millilitern gearbeitet. Die Gelierzeit der Alginatlösung nach Zusatz des Calciumchlorids beträgt lediglich 30 bis 60 Sekunden. Versucht man dieses Verfahren auf größere Maßstäbe zu übertragen, so stellt sich heraus, dass die gewünschte Verzögerung durch Zugabe des Komplexierungsmittels zur Lösung des Natriumalginates nicht ausreichend ist, und ein relativ großformatiges Produkt mit einer hohen Homogenität nicht erhalten werden kann.

Weiterhin ist bei dem genannten Verfahren die Anwendung von oberflächenaktiven Mitteln obligatorisch, um eine ausreichende Dispergierung der Komponenten zu erzielen. Die Verwendung derartiger oberflächenaktiver Mittel kann jedoch zu Unverträglichkeiten beispielsweise bei der Anwendung auf der Haut führen. Die Tatsache, dass in dem Verfahren der US 5718916 keine ausreichende Verzögerung der Fällung durch die vorherige Zugabe des Komplexierungsmittels erzielt wird, wird auch in der GB 2357765 desselben Erfinders bestätigt, worin das Verfahren der US 5718916 folglich als nachteilig beschrieben wird. Die GB 2357765 offenbart ein Verfahren zur Herstellung von wasserunlöslichen Alginatschwämmen oder Schaumprodukten für die Herstellung von Wundpflastern oder chirurgischen Produkten, bei dem ebenfalls wasserlösliches Alginat durch den Zusatz mehrwertiger Metallionen in Anwesenheit eines schaumbildenden Mittels vernetzt wird. Auf die Anwesenheit eines Komplexierungsmittels wird dabei bewusst verzichtet. In einer bevorzugten Variante wird in Gegenwart von Ammoniumhydroxid gearbeitet, um die Viskosität des Calciumalginates herabzusetzen. In den Beispielen wird beispielsweise Calciumsulfat und anschließend eine Säure zugesetzt. Auch dieses Verfahren leidet unter dem Nachteil, dass nach Zusatz der Säure die Bildung des mittels der Caiciumionen vernetzten Alginate relativ schnell verläuft, so dass homogene dicke Schichten nicht erhalten werden können. Außerdem erfordert das Verfahren die Anwesenheit eines Schaumbildners, oberflächenaktiver Mittel, eines Boratpuffers sowie die erwähnten Ammoniumverbindungen. Dies macht das Verfahren schwer steuerbar, und die erhaltenen Produkte enthalten eine Vielzahl von Komponenten, deren physiologische Wirkungen berücksichtigt werden müssen.

Aus der DE 202 19 666 U1 sind Auflagen für dermatologische Anwendungen, enthaltend ein Trägermaterial auf Polymerbasis, insbesondere Alginsäure-Basis, beschrieben. Konkrete Beispiele zur Herstellung dieser Auflagen sind diesem Gebrauchsmuster nicht zu entnehmen.

Weiterhin offenbart die DE 43 28 329 gefriergetrocknete Biomatrices zur Hautbefeuchtung und zur topischen transdermalen Applikation von pharmazeutischen kosmetisch aktiven Wirkstoffen, die natürliche Polysaccharide und modifizierte Polysaccharide enthalten.

Auch erwähnt diese Schrift bereits die Stabilisierung der Biomatrix durch Bildung von Calciumalginatgerüsten durch Zusatz von Calciumionen.

Wie man homogene dickere Alginatschichten herstellt, lässt sich dieser Druckschrift nicht entnehmen.

Die Herstellung kleinformatiger Alginatschwämme für die orale Einnahme durch Versetzen von Na-Alginatlösung mit einem löslichen Calciumsalz (Calciumgluconat) ist in der WO 01/17377 beschrieben.

Dieses Verfahren ist jedoch für die Herstellung großformatiger Alginatschwämme aus den vorstehend bereits erwähnten Gründen (keine homogene Einarbeitung der Calciumionen) ungeeignet. Durch die entstehenden Inhomogenitäten ist weiterhin die dort vorgeschlagene Beaufschlagung mit Wirkstoffen erschwert.

Aus der WO94/00512 ist ein Verfahren zur Bildung von Polysaccharidschäumen bekannt, insbesondere auf Alginatbasis. Diese Patentschrift offenbart in einer Ausführungsform auch die Variante, bei der ein unlösliches Carbonat oder Hydrogencarbonatsalz von mehrwertigen Metallkationen in dem geschäumten Polysaccharid dispergiert werden, und der Schaum nachfolgend mit einer starken Säure behandelt wird, um Kohlendioxid freizusetzen, und durch die sich bildenden Kationen das Polysaccharid unter Bildung einer dimensionsstabilen Schaumstruktur zu vernetzen. Auf diese Weise lassen sich nach Angaben in der Druckschrift Schaumdicken von bis zu 5 mm stabilisieren. Diese Dicken sind

jedoch insbesondere dann unzureichend, wenn es gewünscht ist, die Schaumformkörper anschließend in dünnere Schichten zu schneiden. Außerdem führt die Gasbildung während der Herstellung dazu, dass sich die Porengrößen kaum kontrollieren lassen und starke Inhomogenitäten im Schaum resultieren.

Ein weiteres Verfahren zur Herstellung von Alginatschwämmen ist aus der US 3653383 bekannt. Dabei wird zunächst Ca-Alginat aus Alginsäure und Calciumcarbonat hergestellt, das entstandene Ca- Alginat anschließend zerkleinert und das resultierende Gel der Gefriertrocknung unterworfen. Auf diese Weise können zwar relativ großformatige schwammartige Materialien hergestellt werden, die erhaltenen Produkte zerfallen jedoch in Wasser relativ rasch. Die Alginatschwämme besitzen daher-insbesondere auch wenn sie in dünne Schichten geschnitten werden-eine für kosmetische oder medizinische Auflagen unzureichende Nassfestigkeit, insbesondere Nassreißfestigkeit.

Die Aufgabe der vorliegenden Erfindung bestand somit in der Bereitstellung von relativ großformatigen, hoch homogenen Formkörpern auf der Basis von Verbindungen zwischen Alginaten und mehrwertigen Metallionen, die über eine hohe Nassfestigkeit, insbesondere über eine hohe Nassreißfestigkeit verfügen, die mit üblichen Schneidvorrichtungen in dünne Schichten geschnitten werden können, die optisch ansprechend sind, d. h. insbesondere einen hohen Weißgrad aufweisen, und die somit in kosmetischen oder medizinischen Anwendungen, wie als kosmetische Hautauflage oder als medizinische Wundauflage etc. verwendet werden können. Weiterhin sollte es die Bereitstellung homogener dicker poröser Alginat-Schichten ermöglichen, aus denen in einfacher Weise durch Komprimieren und/oder Ausstanzen geeignete, auch oral verabreichbare kosmetische oder medizinische Applikationsformen, wie z. B. Implantat-Formkörper, Sättigungskomprimate, Mittel zur kontrollierten, insbesondere verzögerten Wirkstofffreisetzung oder dergleichen herzustellen.

Den Erfindern der vorliegenden Patentanmeldung gelang es überraschend, homogene, relativ dicke, großformatige Formkörper auf der Basis von Alginaten mehrwertiger Metallsalzen bereitzustellen, die durch spezielle, den Gegenstand dieser Erfindung bildende Verfahren erhalten werden können, welche die obigen Probleme der Formkörper aus dem Stand der Technik lösen, und die sich daher hervorragend für die Herstellung kosmetischer oder medizinischer Produkte eignen.

## DETAILLIERTE BESCHREIBUNG DER ERFINDUNG

Die vorliegende Patentanmeldung stellt somit ein Verfahren zur Herstellung von Alginat-haltigen porösen Formkörpern bereit, dass die Schritte umfasst:

a) Herstellen einer wässrigen Lösung eines wasserlöslichen Alginates,

b1) Zugabe eines oder mehrerer Salze eines mehrwertigen Metallions mit einem mehrzähnigen komplexierenden Anion zur wässrigen Lösung des wasserlöslichen Alginates, und Verschiebung des Komplexbildungsgleichgewichtes des mehrwertigen Metallions und des mehrzähnigen komplexierenden Anions unter Erhöhung der verfügbaren Konzentration des mehrwertigen Metallions in der Alginat-Lösung, und dadurch Bildung von Salzen des Alginates mit dem genannten mehrwertigen Metallion,

c) Gießen der (noch) fließfähigen wässrigen Alginatzusammensetzung in eine Form,

d) Trocknung der wässrigen Alginat-Zusammensetzung unter Bildung des porösen, Alginat-haltigen Formkörpers

wobei die Verschiebung des Komplexbildungsgleichgewichtes des mehrwertigen Metallions und des mehrzähnigen komplexierenden Anions in Schritt b1) durch den Zusatz mindestens einer Säure erfolgt.

Die vorliegende Patentanmeldung offenbart ferner ein Verfahren zur Herstellung von Alginat-haltigen porösen Formkörpern, umfassend die Schritte:

Schritt a)

Die in Schritt a) verwendeten wasserlöslichen Alginate sind bevorzugt Alkalimetallalginate, wie Alginate von Natrium, Kalium etc.

Die zugrundeliegende Alginsäure ist ein natürliches saures Polysaccharid, das vor allem aus sogenannten Braunalgen (Phaecophyceae) mit einem hohen, von etwa 30 000 bis 200 000 Dalton schwankenden Molekulargewicht extrahiert wird und Ketten enthält, die aus D-Mannuron-und L-Guluronsäure gebildet werden. Der Polymerisationsgrad ändert sich in Abhängigkeit von der Art der zur Extraktion verwendeten Alge, der Jahreszeit, in der die Algen gesammelt wurden, und dem Ursprungsort der Algen, sowie dem Alter der Pflanzen. Die Hauptarten der Braunalgen, aus denen Alginsäure erhalten wird, sind beispielsweise Macrocystis pyrifera, Laminaria cloustoni, Laminaria hyperborea, Laminaria flexicaulis, Laminaria digitata, Ascophyllumnodosum und Fucus serratus. Alginsäure oder alkalische Alginate können

jedoch auch mikrobiologisch, beispielsweise durch Fermentation mit Pseudomonas aeruginosa oder Mutanten von Pseudomonas putida, Pseudomonas fluorescens oder Pseudomonas mendocina, erhalten werden (s. z. B. EP-A-251905 und Römpp Chemie Lexikon"Naturstoffe"Thieme Verlag, 1997 und dort zitierte Dokumente).

Erfindungsgemäß bevorzugt sind Alginate mit einer durchschnittlichen Partikelgrösse von bis zu etwa 0,2 mm und einer Viskosität in wässriger Lösung (1-% ige Lösung, pH 7, 20 °C von 300 bis 800 mPas).

Erfindungsgemäß besonders bevorzugt ist Natriumalginat.

Die in Schritt a) verwendete wässrige Lösung des wasserlöslichen Alginates weist bevorzugt eine solche Konzentration auf, dass sich in der nach Schritt b) gebildeten wässrigen Suspension eine Konzentration von 0,2 bis 3,0 %, bevorzugter 0,3 bis 2,5-%, noch bevorzugter 0,4 bis 1,2% (w/w) Alginat bezogen auf die eingesetzte Wassermenge einstellt.

Die Lösung kann hergestellt werden durch Suspendieren der gewünschten Alginatmenge beispielsweise in destilliertem Wasser. Die Konzentration des Alginates in der wässrigen Suspension hat Einfluss auf die Härte der gebildeten porösen Formkörper. Konzentrationen von mehr als 2% (w/w) führen zu relativ harten bzw. spröden Formkörpern, was weniger bevorzugt ist. Kleinere Konzentrationen als 2 % (w/w) führen zu weniger spröden Formkörpern, was bevorzugter ist.

Schritt b 1)

Im Schritt b1) werden ein oder mehrere Salze, bevorzugt ein Salz eines mehrwertigen Metallions mit einem mehrzähnigen komplexierenden Anion zur wässrigen Lösung des wasserlöslichen Alginates, die in Schritt a) erhalten wird, gegeben.

Mehrwertige Metallionen sind zweckmäßig solche, die mit dem verwendeten Alginat schwerlösliche Verbindungen bilden, d. h. als vernetzende Metallionen wirken.

Solche mehrwertigen Metallionen schließen beispielsweise Ionen von Erdalkalimetallen und Übergangsmetallen ein, die mit Alginaten schwerlösliche Verbindungen bilden. Erdalkalimetallionen, wie z. B. Beryllium, Magnesium oder Calcium sind bevorzugt. Besonders bevorzugt ist Calcium. Beryllium und Magnesium sind weniger bevorzugt, da ersteres kosmetisch nicht akzeptabel ist und die Vernetzungswirkung des Magnesiums gering ist. Daher sind Calciumsalze erfindungsgemäß besonders bevorzugt, da sie physiologisch, insbesondere kosmetisch akzeptabel sind und eine hohe Vernetzungs-bzw. Gelbildungswirkung gegenüber Alginaten aufweisen.

Weiterhin können beispielsweise auch Barium, Strontium, Zink, Mangan, Eisen, Aluminium etc. verwendet werden.

Das mehrzähnige, komplexierende Anion in dem Komplexsalz des mehrwertigen Metollions ist erfindungsgemäß bevorzugt ein Carboxylat einer Polycarbonsäure. Bevorzugt sind Carboxylate aliphatischer Di-bis Tetracarbonsäuren, wie beispielsweise Zitronensäure (2-Hydroxy-1,2, 3-Propantricarbonsäure), Äpfelsäure, Oxalsäure, 1,3- Propandicarbonsäure, Agaricinsäure, Ethylendiamintetraessigsäure (EDTA), 1,2, 3,-Propantricarbonsäure etc.

Besonders bevorzugt sind physiologisch verträgliche, insbesondere hautverträgliche Polycarbonsäuren. Dazu zählen insbesondere die Carboxylate von $\alpha$-Hydroxypolycarbonsäuren, wie Zitronensäure.

Als mehrzähnige komplexierende Anionen sind Zitrat, Malat und das Anion von EDTA besonders bevorzugt. Am meisten bevorzugt sind Zitrate.

Erfindungsgemäß ist das in Schritt b1) zugegebene Komplexsalz eines mehrwertigen Metallions mit einem mehrzähnigen komplexierenden Anion besonders bevorzugt Calciumcitrat (Stöchiometrie: $Ca_3Citrat_2$).

Die Zugabe des Komplexsalzes eines mehrwertigen Metallions mit einem mehrzähnigen komplexierenden Anion in Schritt b1) kann durch Zusatz in fester oder in gelöster Form erfolgen.

Die Zugabe des Komplexsalzes zur Alginatlösung erfolgt zweckmäßigerweise in einem Temperaturbereich zwischen 5 und 80° C, bevorzugt jedoch bei Raumtemperatur (20° C).

Die Menge des im Schritt b1) zugegebenen Komplexsalzes wird zweckmäßig so gewählt, dass die Konzentration des Komplexsalzes in der resultierenden Lösung etwa 0,1 bis 500 mmol/Liter beträgt.

Die Menge des zugegebenen Komplexsalzes bezogen auf die Menge des Alginates in der Lösung wird bevorzugt so gewählt, dass das molare Verhältnis des Komplexsalzes und des Alginates etwa 0,001 bis 0,1 beträgt.

Im Anschluss an die Zugabe des Komplexsalzes erfolgt die Verschiebung des Komplexbildungsgleichgewichtes des mehrwertigen Metallions und des mehrzähnigen komplexierenden Anions in der wässrigen Lösung des Alginates:

$$[\text{Metallion (aq.)}]^{x+} + [\text{Komplexbildendes Anion}]^{x-} \rightleftarrows [\text{Komplex}]$$

wobei selbstverständlich auch ungleiche Ladungen von Metallion und komplexbildendem Anion mit entsprechende Stöchiometrie möglich sind, wie im System Calciumzitrat.

Dieses Gleichgewicht wird üblicherweise durch die sogenannte Komplexbildungskonstante beschrieben:

$$K_a = \frac{[Komplex]}{[Me^+] \cdot [A^-]}$$

worin $[Me^+]$, $[A^-]$ und [Komplex] jeweils die Konzentrationen (Aktivitäten) des mehrwertigen Metallions, des komplexierenden mehrzähnigen Anions und des Komplexes in der Lösung sind. Die Komplexbildungskonstante ist der Kehrwert der Dissoziationskonstante.

Die Komplexbildungskonstante gibt Aufschluss über die Stabilität des Komplexes in der jeweiligen chemischen Umgebung und wird deshalb auch als Stabilitätskonstante des Komplexes bezeichnet. Je größer der Wert der Konstante ist, desto stabiler ist auch der Komplex.

Die erfindungsgemäß durchgeführte Verschiebung des o. g. Gleichgewichtes in Schritt b1) erfolgt beispielsweise durch Verringerung der Konzentration des komplexbildenden Anions in der Lösung. Dadurch wird gemäß der Gleichgewichtskonstante die Konzentration des unkomplexierten mehrwertigen Metallion in der Lösung erhöht. Die Verschiebung des Gleichgewichtes kann beispielsweise, da die Gleichgewichtskonstante unter anderem temperaturabhängig sein kann, auch durch eine Veränderung, insbesondere ErhöhungderTemperaturerfolgen. Auch ist der Zusatz eines anderen Metallsalzes denkbar, welches die Gleichgewichtsreaktion komplexiertes Anion/freies Anion beeinflusst, ohne jedoch unlösliche Alginate zu bilden.

Bevorzugt erfolgt die Verschiebung des Gleichgewichtes jedoch über eine Verringerung der Konzentration des freien komplexierenden Anions in der Lösung, besonders bevorzugt durch den Zusatz mindestens einer Säure:

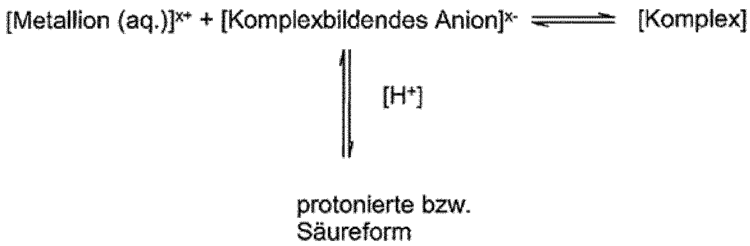


Die zugesetzte Säure ist bevorzugt eine stärkere Säure als die konjugierte Säure des komplexbildenden Anions, vermag dieses also zu protonieren. Es kann aber auch die konjugierte Säure selbst wie beispielsweise Zitronensäure im Falle von Zitrat als Anion zugesetzt werden. Da das Zitrat, das aus der Dissoziation des Salzes des mehrwertigen Kations, wie z. B. $Ca^{2+}$ resultiert, als $Zitrat^{3-}$ gebildet wird.

wird es in wässriger Lösung durch Zusatz von Zitronensäure unter Bildung von Hydrogenzitraten protoniert, und mithin dem Komplexbildungsgleichgewicht entzogen.

Zum Beispiel:

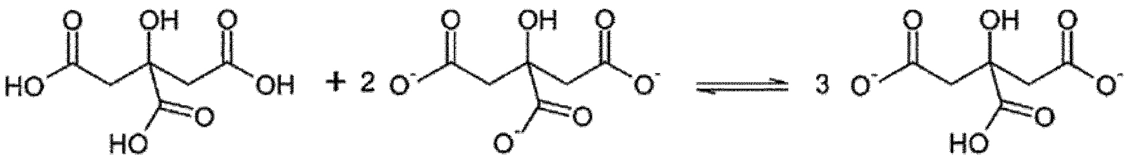

Bevorzugte Säuren sind beispielsweise anorganische Mineralsäuren, wie z. B. Salzsäure, Schwefelsäure, Phosphorsäure oder aliphatische Carbonsäuren, wie z. B. Essigsäure etc.

Die Menge der zugesetzten Säure hängt von dem verwendeten Komplexsalz und dessen Komplexbildungskonstante in wässriger Lösung ab. Sie kann beispielsweise etwa das 0,1-20-fache (mol/mol) der Konzentration des Komplexsalzes betragen. Insbesondere beträgt das molare Verhältnis von Calciumcitrat zu einer Säure, wie z. B. Zitronensäure, bevorzugt von 0,1 bis 20, bevorzugter von 0,5 bis 10.

In der Regel reicht die Einstellung des pH-Wertes der Lösung auf weniger als etwa 6,0 aus, um die Komplexbildungskonstante soweit zu verschieben, dass die Konzentration des mehrwertigen Metallsalzes soweit erhöht ist, dass das Löslichkeitsprodukt des Alginatsalzes überschritten wird, d. h. das unlösliche Alginat des mehrwertigen Metallsalzes ausfällt bzw. die Lösung geliert.

Es wurde überraschend gefunden, dass der pH-Wert, der in diesem Schritt eingestellt wird, einen Einfluss auf die Reißfestigkeit der erhaltenen porösen Formkörper besitzt. Um eine höhere Reißfestigkeit zu erzielen, sind pH-Werte

von weniger als 6 bevorzugt, bevorzugter von weniger als 5. Diese niedrigen pH-Werte sind besonders bevorzugt in Kombination mit einer niedrigen Alginatkonzentration von weniger als 2 % (w/w) in der Gesamtsuspension, die sich in Schritt b) einstellt.

Durch Menge und Geschwindigkeit des Zusatzes der Säure sowie gegebenenfalls durch Steuerung der Temperatur kann die Geschwindigkeit der Bildung des unlöslichen Alginats, mithin die Fließfähigkeit bzw. Gießfähigkeit der Alginat-Lösung bzw. -Suspension sehr genau und bequem gesteuert werden, insbesondere aufgrund der hohen Diffusionsgeschwindigkeit der Protonen in der wässrigen Alginatlösung. Dadurch ist es möglich, nach Trocknung homogene Formkörper hoher Dicken von mindestens etwa 1 cm zu erhalten, die über eine ausreichend hohe Nassfestigkeit, insbesondere Nassreißfestigkeit verfügen, so dass sie gegebenenfalls nach nachfolgendem Schneiden in dünnere Schichten oder Komprimieren und/oder Ausstanzen, wie unten beschrieben, als kosmetische oder medizinische schwammförmige nassfeste Materialien verwendet werden können.

Schritt b2)

Eine weitere Ausführungsform (Schritt b2)) des erfindungsgemäßen Verfahrens wird offenbart, in der die gegenüber dem Stand der Technik weitere Verzögerung der Bildung des unlöslichen Alginates in der Alginatlösung erfolgt, die ein homogeneres Einarbeiten des mehrwertigen Metallsalzes in der Lösung des Alginats und damit eine homogene Beschaffenheit des porösen Formkörpers ermöglicht, dadurch, dass man der Alginatlösung aus Schritt a) nicht wie Im Stand der Technik ein lösliches Salz eines mehrwertigen Metallions, das mit Alginaten schwerlösliche Salze bildet, wie z. B. Calciumchlorid, sondern schwerlösliche Salze dieser mehrwertigen Metallionen, wie z. B. $CaSO_4$ zusetzt.

In Schritt b2) wird zunächst ein mehrzähniger Komplexbildner für ein mehrwertiges Metallion zur wässrigen Lösung des wasserlöslichen Alginats gegeben. Der mehrzähnige Komplexbildner wird naturgemäß in der Form einer ionischen Verbindung oder als kovalente Verbindung, wie in der Form der konjugierten Säure zugegeben. Der mehrzähnige Komplexbildner kann in fester oder gelöster Form zur Lösung des Alginates zugegeben werden. Bei dem genannten Komplexbildner kann es sich im Prinzip sowohl um Salze mehrwertiger Metallionen, die schwerlösliche Alginate bilden als auch um Salze ein-oder mehrwertiger Metallionen, die mit Alginaten keine schwerlöslichen Verbindungen bilden handeln. Auch Mischungen solcher Metallsalze sind anwendbar. Bevorzugt sind Salze ein-oder mehrwertiger Metallionen, die mit Alginaten keine schwerlöslichen Verbindungen bilden, wie z. B. Natriumzitrat, oder deren konjugierte Säuren, wie z. B. Zitronensäure, da dann die retardierende Wirkung des mehrzähnigen Anions auf die Bildung freier mehrwertiger Metallionen, die zur Bildung schwerlöslicher Alginate dienen können, erhöht ist. Grundsätzlich können aber auch die in Schritt b1) verwendeten Salze mehrwertiger Metallionen mit mehrzähnigen komplexierenden Anionen, wie z. B. Calciumzitrat zugesetzt werden.

In dieser und den anderen Varianten setzt man gegebenenfalls nach oder während des Zumischen des schwerlöslichen Metallsalzes eines mehrwertigen Metallions, wie z. B. Calciumsulfat, noch Säure zu, um die Konzentration freier mit Alginaten schwer lösliche Verbindung bildender Metallionen zu erhöhen und die homogene Vernetzung des Alginates zu beschleunigen. Bevorzugte Säuren sind dabei beispielsweise anorganische Mineralsäuren, wie z. B. Salzsäure, Schwefelsäure, Phosphorsäure oder aliphatische Carbonsäuren, wie z. B. Essigsäure etc. Besonders bevorzugt ist Salzsäure.

Auch in der Variante des Schrittes b2) zeigt sich, dass der eingestellte pH-Wert, einen Einfluss auf die Reißfestigkeit der erhaltenen porösen Formkörper besitzt. Um eine höhere Reißfestigkeit zu erzielen, ist daher auch in Schritt b2) ein pH-Wert von weniger als 6 bevorzugt, bevorzugter von weniger als 5.

Diese niedrigen pH-Werte sind wiederum besonders bevorzugt in Kombination mit einer niedrigen Alginatkonzentration von weniger als 2 % (w/w), die sich in Schritt b) in der Gesamtsuspension einstellt. Die Einstellung des pH-Wertes kann prinzipiell auch durch vorherige Zugabe einer Säure, wie HCl, zur Alginatlösung bzw. zur Lösung aus Alginat, versetzt mit dem Komplexbildner, wie Natriumzitrat oder Zitronensäure, und anschließender Zugabe des schwerlöslichen Metallsalzes, wie $CaSO_4$ erfolgen.

Die Konzentration des zugesetzten mehrzähnigen Komplexbildners für ein mehrwertiges Metallion beträgt etwa von 0,0001 bis 1 mol/Liter, bevorzugt von 0,001 bis 0,5 mol/Liter. Das molare Verhältnis der Menge des wassserlöslichen Alginates bezogen auf die molare Menge des zugesetzten mehrzähnigen Komplexbildners für ein mehrwertiges Metallion beträgt bevorzugt etwa von 0,0001 bis 1, bevorzugter von 0,001 bis 0,5.

Hinsichtlich der mehrwertigen Metallionen, die in Form ihrer schwerlöslichen Salze im zweiten Zugabeschritt des Schritts b2) zugesetzt werden, handelt es sich um solche, die mit Alginaten schwerlösliche Salze bzw. vernetzte Alginate bilden, und diesbezüglich kann daher zu den unter "Schritt b1)" genannten Salzen verwiesen werden. Die Wahl der entsprechenden Anionen ist an sich beliebig, sie müssen jedoch mit den mehrwertigen Metallionen bzw. Kationen in Wasser schwer lösliche Salze bilden. Bevorzugt werden auch hier Calciumsalze, insbesondere Calciumsulfat. $CaCO_3$ ist wie andere Carbonate auch weniger bevorzugt, da sich unter den bevorzugt sauren Bedingungen der Herstellung des schwerlöslichen Alginates $CO_2$ bilden kann, was die Steuerung der Reaktion bzw. die Beschaffenheit des Alginathaltigen porösen Formkörpers erschwert.

Bevorzugt beträgt die Löslichkeit des in Schritt b2) zugemischten, in Wasser schwerlöslichen Salzes des mehrwertigen Metallions in Wasser bei 20°C weniger als 10 g/Liter, bevorzugter weniger als 5 g/Liter, noch bevorzugter von 0,1 bis 3 g/Liter. Ist die Löslichkeit höher, kann es zur rascheren Bildung der schwerlöslichen Alginate kommen, was zu einer Verringerung der möglichen Verarbeitungsdauer und damit zu einem inhomogeneren Produkt führt. Ist die Löslichkeit geringer als der oben erwähnte Bereich, kann die Bildung der schwerlöslichen bzw. vernetzten Alginate zu langsam sein, was ebenfalls nicht erwünscht ist.

Durch das Zumischen von weiteren Salzen, insbesondere solchen, die keine schwerlöslichen Alginate bilden, wie z. B. Natriumsulfat, Natriumchlorid etc. kann die Löslichkeit der in Wasser schwerlöslichen Salze mehrwertiger Metallionen noch weiter verringert werden und damit die Verarbeitbarkeit bzw. Homogenisierbarkeit weiter erhöht werden.

Die Menge des schwerlöslichen Salzes des mehrwertigen Metallions wird zweckmäßig so gewählt, dass die Konzentration des Salzes in der resultierenden Lösung von etwa 0,1 bis 500 mmol/Liter beträgt, wobei hier die Gesamtmenge des Salzes bezogen auf das Volumen der Lösung gemeint ist, auch wenn sich das Salz nicht vollständig löst.

Die Menge des zugegebenen schwerlöslichen Salzes des mehrwertigen Metallions bezogen auf die Menge des löslichen Alginates in der Lösung wird bevorzugt so gewählt, dass das molare Verhältnis des Alginates zum schwerlöslichen Salz des mehrwertigen Metallions von 0,001 bis 1 beträgt.

Die Menge des zugegebenen schwerlöslichen Salzes des mehrwertigen Metallions bezogen auf die Menge des vorgelegten mehrzähnigen Komplexbildners in der Lösung wird bevorzugt so gewählt, dass das molare Verhältnis des schwerlöslichen Salzes des mehrwertigen Metallions und des mehrzähnigen Komplexbildners Anions von 0,1 bis 10 beträgt.

Die Bildung der schwerlöslichen Alginate wird nach beiden Verfahrensvarianten b1) und b2) zweckmässig so gesteuert, dass die Erhöhung der Konzentration des unkomplexierten zweiwertigen Metallions so gering ist, dass mindestens etwa 1 Minute, bevorzugt etwa 2 Minuten noch bevorzugter mindestens etwa 3 Minuten eine Fliessfähigkeit der Alginatlösung ausgedrückt als Viskosität bei Raumtemperatur (20° C) von unter etwa 1000 mPas ermöglicht wird.

Die Bildung der Alginatgele bzw. das Mischen gemäss der Schritte b1) und b2) wird bevorzugt in Mischern mit einem Stator-/Rotorsystem durchgeführt, z. B. in einer Kolloidmühle.

Schritt c)

Das Giessen der (noch) fließfähigen Alginatzusammensetzung in eine für die spätere Trocknung gewünschte Form kann in an sich bekannter Weise erfolgen. Dabei sind Schichtdicken der fließfähigen Alginatzusammensetzung von bis zu etwa 50 cm möglich. Bevorzugte Formen sind Kastenformen mit rechteckigem Grundriss. Das Giessen kann in jeder geeigneten Stufe des Verfahrens erfolgen. So kann bereits die Lösung des wasserlöslichen Alginates aus Stufe a) in die spätere für die Trocknung verwendete Form gegossen werden, wenn eine ausreichende Durchmischung in dieser Form gewährleistet werden kann. Bevorzugt erfolgt jedoch das Giessen nach dem die Vernetzung bzw. Fällung des schwerlöslichen Alginates in Schritt b1) oder b2) in Gang gesetzt wurde.

Schritt d)

Die Trocknung in Schritt d) erfolgt in an sich bekannter Weise.

Besonders bevorzugt ist die Gefriertrocknung. Diese kann ebenfalls in an sich bekannter Weise erfolgen, und hier kann beispielsweise verwiesen werden auf die DE 4328329 C2 oder die DE 4028622 C2, auf die bezüglich des Schrittes d) des erfindungsgemäßen Verfahrens ausdrücklich Bezug genommen werden soll und die somit Teil des erfindungsgemäßen Verfahrens sind.

In einer bevorzugten Ausführungsform des erfindungsgemäßen Verfahrens erfolgt zusätzlich vor dem Schritt d), insbesondere vor Schritt c) das Zugeben mindestens einer weiteren Komponente, ausgewählt aus der Gruppe, die besteht aus: kosmetischen oder medizinischen Wirkstoffen, weiteren natürlichen oder synthetischen Hydrokolloidbildenden Polymeren und kosmetischen oder medizinischen Hilfs-bzw. Zusatzstoffen.

Weitere natürliche oder synthetische Hydrokolloid-bildende Polymere schließen (teilweise) wasserlösliche, natürliche oder synthetische Polymere ein, die in wässrigen Systemen Geie bzw. viskose Lösungen bilden. Sie werden zweckmäßig ausgewählt aus weiteren natürlichen Polysacchariden, synthetisch modifizierten Derivaten davon oder synthetischen Polymeren. Weitere Polysaccharide schließen beispielsweise Homoglykane oder Heteroglykane ein, wie zum Beispiel Carrageen, Pektine, Tragant, Guar-Gummi, Johannisbrotkernmehl, AgarAgar, GummiArabikum, Xanthan, natürliche und modifizierte Stärken, Dextrane, Dextrin, Maltodextrine, Chitosan, Glucane, wie ß-1, 3-Glucan, ß-1, 4-Glucan, wie Cellulose, Mucopolysaccharide, wie insbesondere Hyaluronsäure etc. Synthetische Polymere schließen beispielsweise ein: Celluloseether, Polyvinylalkohol, Polyvinylpyrrolidon, synthetische Cellulosederivate, wie Methyilcellulose, Carboxycellulose, Carboxymethylcellulose, insbesondere Natriumcarboxymethylcellulose, Celluloseester, Cellulosesether wie Hydroxypropylcellulose, Polyacrylsäure, Polymethacrylsäure, Poly (methylmethacrylat) (PMMA),

Polymethacrylat (PMA), Polyethylenglykole etc. Es können auch Mischungen dieser Polymere verwendet werden. Die von Hydrokolloid- bildenden Proteinen, wie z. B. Kollagen ist jedoch nicht bevorzugt, da einige Endverbraucher zunehmend die Anwendung rein pflanzlicher Produkte insbesondere in der Kosmetik vorziehen.

Erfindungsgemäß besonders bevorzugt wird Hyaluronsäure und/oder deren Salze und/oder deren Derivate zusätzlich hinzugesetzt. Hyaluronsäure ist ein hochviskoses natürliches Glucosaminoglycan mit alternierenden $ß_{1-3}$ Glucoronsäure und $ß_{1-4}$-Glucosaminanteilen; ihr Molekulargewicht liegt zwischen 50000 und einigen Millionen Da.

Hyaluronsäure wird häufig als Natriumsalz, z. B. bei der Therapie, hauptsächlich in der Ophthalmologie, Chirurgie und der Kosmetik verwendet. Die Salze der Hyaluronsäure, die mit Alkali-, Erdalkali, Magnesium-, Aluminium-, Ammonium-oder substituierten Ammoniumionen gebildet werden, können als Träger zur Steigerung der Absorption von Arzneimitteln verwendet werden (s. z. B. Römpp Chemie Lexikon "Naturstoffe" Thieme Verlag, 1997 und dort zitierte Dokumente).

Erfindungsgemäß besonders bevorzugt ist Natriumhyaluronat mit einem Molekulargewicht von etwa 1.000. 000 bis 2.500. 000 Dalton. Der Zusatz der Hyaluronsäure zu dem erfindungsgemäßen Verfahren führt insbesondere in der Verfahrensvariante b1) aber auch in der Verfahrensvariante b2) völlig überraschend zu einem erhöhten Weißgrad der erhaltenen Alginat-haltigen porösen Formkörper. Dies ist insbesondere in der kosmetischen Anwendung aus ästhetischen Gründen sehr bevorzugt. Darüber hinaus entfaltet die Hyaluronsäure aber auch insbesondere bei der topischen bzw. äußeren Anwendung ihre therapeutische Wirkung wie z. B. die Befeuchtung der Haut oder die Förderung der Wundheilung.

Die Hyaluronsäure oder deren Salze werden den erfindungsgemäßen Alginat-haltigen porösen Formkörpern in einer Menge von etwa 0,1 bis 90 Gew.-%, bevorzugt 1 bis etwa 70 Gew.-%, bezogen auf den getrockneten Formkörper, zugesetzt.

In einer weiteren bevorzugten Ausführungsform enthalten die erfindungsgemäßen porösen Formkörper Carboxymethylcellulose, insbesondere Natriumcarboxymethylcellulose. Der Zusatz von Natriumcarboxymethylcellulose führt überraschend zu einer Verbesserung der optischen Dichte der erfindungsgemäßen porösen Formkörper ohne dabei die Härte oder die Sprödheit der Formkörper zu erhöhen. Im Gegenteil führt der Zusatz von Natriumcarboxymethylcellulose, zu einer Verbesserung der Flexibilität der erhaltenen porösen Formkörper. Weiterhin führt der Zusatz der Carboxymethylcellulose, insbesondere Natriumcarboxymethylcellulose, zu einer Stabilisierung der Formkörper. Bei der Herstellung Carboxymethylcellulose-haltiger Formkörper verhindert die Carboxymethylcellulose, insbesondere Natriumcarboxymethylcellulose, überraschend auch eine Sedimentation des schwerlöslichen Salzes, insbesondere des $CaSO_4$. Die Carboxymethylcellulose, insbesondere Natriumcarboxymethylcellulose, kann in den erfindungsgemäßen Formkörpern in einer Menge von bis zu 90 Gew.-% bezogen auf den Trockengehalt des Formkörpers vorliegen. Dies entspricht einzustellenden Vorzugsbereichen in der wässrigen Suspension von etwa bis zu 3 Gew. - %, bevorzugt 0,2 bis 3 Gew.-%.

Eine bevorzugte Ausführungsform des erfindungsgemäßen Formkörpers enthält Carboxymethylcellulose, insbesondere Natriumcarboxymethylcellulose, und Hyaluronsäure bzw. deren Salze oder Derivate. Erfindungsgemäß zugesetzte Wirkstoffe schließen insbesondere kosmetische oder therapeutische bzw. pharmazeutische, insbesondere für die äußere Anwendung geeignete Wirkstoffe ein. Bevorzugt enthält der erfindungsgemäß hergestellte Formkörper mindestens einen kosmetischen und/oder pharmazeutischen Wirkstoff. Dementsprechend handelt es sich bei den erfindungsgemäß bevorzugten Formkörpern bevorzugt um kosmetische oder therapeutische Mittel. Kosmetische Formkörper bzw. unter Verwendung kosmetischer Wirkstoffe hergestellte Formkörper im Sinne der Erfindung sind im wesentlichen Mittel im Sinne des Lebensmittel- und Bedarfsgegenständegesetzes (LMBG), d. h., Stoffe oder Zubereitungen aus Stoffen, die dazu bestimmt sind, äußerlich am Menschen zur Reinigung, Pflege, oder zur Beeinflussung des Aussehens oder des Körpergeruchs, oder zur Vermittlung von Geruchseindrücken angewendet zu werden, es sei, denn, dass sie überwiegend dazu bestimmt sind, Krankheiten, Leiden, Körperschäden oder krankhafte Beschwerden zu lindern oder zu beseitigen. In diesem Sinne handelt es sich bei den erfindungsgemäß hergestellten kosmetischen Formkörpern beispielweise kosmetische Auflage, wie z. B. Gesichtsmasken etc., die beispielweise als Hautwasch- und reinigungsmittel, Hautpflegemittel, insbesondere Gesichtshautpflegemittel, Augenkosmetika, Lippenpflegemittel, Nagelpflegemittel, Fusspflegemittel sowie als Haar- oder Zahnpflegemittel dienen können.

Beispiele kosmetischer gegebenenfalls auch z. B. dermatologischer, therapeutisch wirksamer Verbindungen schließen ein : Antiaknemittel, antimikrobielle Mittel, Antitranspirationsmittel, adstringierende Mittel, desodorierende Mittel, Enthaarungsmittel, Konditionierungsmittel für die Haut, hautglättende Mittel, Mittel zur Steigerung der Hauthydratation wie z. B. Glycerin oder Harnstoff, Sonnenschutzmittel, Keratolytika, Radikalfänger für freie Radikale, antiseptische Wirkstoffe, Wirkstoffe zur Behandlung der Anzeichen der Hautalterung und/oder Mittel, welche die Differenzierung und/oder Proliferation und/oder Pigmentierung der Haut modulieren, Vitamine wie Vitamin C, Wirkstoffe mit reizender Nebenwirkung, wie alpha-Hydroxysäuren, ß-Hydroxysäuren, alpha- Ketosäuren, ß -Ketosäuren, Retinoide (Retinol, Retinal, Retin-Säure) Anthrolinen (Dioxyanthranol), Anthranoide, Peroxide (insbesondere Benzoylperoxid), Minoxidil, Lithiumsalze, Antimetabolite, Vitamin D und seine Derivate; Katechine, Flavonoide, Ceramide, Fettsubstanzen, wie Mineralöle, wie Paraffinöle oder Vaselineöle, Siliconöle, Pflanzenöle wie Kokosöl, Süssmandelöl, Aprikosenöl, Maisöl, Jojobaöl, Olivenöl,

Avocadoöl, Sesamöl, Palmöl, Eukalyptusöl, Rosmarinöl, Lavendelöl, Kiefernöl, Thymianöl, Minzöl, Kardamomöl, Orangenblütenöl, Sojaöl, Kleeöl, Reisöl, Rapsöl und Rizinusöl, Weizenkeimöl und daraus isoliertes Vitamin E, Nachtkerzenöl, Pflanzenlecithine (z. B. Sojalecithin), aus Pflanzen isolierte Sphingolipide/Ceramide, tierische Öle oder Fette, wie Talg, Lanolin, Butteröl, Fettsäureester, Ester von Fettalkoholen und Wachse mit einem der Hauttemperatur entsprechenden Schmelzpunkt (tierische Wachse, wie Bienenwachs, Carnaubawachs und Candelillawachs, mineralische Wachse, wie mikrokristalline Wachse, und synthetische Wachse, wie Polyethylen-oder Silikonwachse), sowie sämtliche für kosmetische Zwecke geeigneten Öle, wie beispielsweise in der CTFA-Abhandlung, Cosmetic Ingredient Handbook, 1. Aufig., 1988, The Cosmetic, Toiletry and Fragrance Association, Inc., Washington, erwähnt, mehrfach ungesättigte Fettsäuren, essentielle Fettsäuren (z. B. gamma-Linolensäure), Enzyme, Coenzyme, Enzymhemmstoffe, hydratisierende Mittel, hautberuhigende Mittel, Detergentien oder schaumbildende Mittel, und anorganische oder synthetische mattierende Füllstoffe, abrasive Mittel.

Weiterhin können Pflanzenwirkstoffextrakte bzw. daraus gewonnene Auszüge oder Einzelstoffe erwähnt werden, die den erfindungsgemäß hergestellten porösen Formkörpern zugesetzt werden können. Allgemein wird der Pflanzenwirkstoffextrakt in der Regel ausgewählt aus der Gruppe bestehend aus festen Pflanzenextrakten, flüssigen Pflanzenextrakten, hydrophilen Pflanzenextrakten, lipophilen Pflanzenextrakten, einzelnen Pflanzeninhaltsstoffen ; sowie deren Mischungen, wie Flavonoide und ihre Aglyka : Rutin, Quercetin, Diosmin, Hyperosid, (Neo) hesperidin, Hesperitin, Ginkgo Biloba (z. B. Ginkoflavonglykoside), Crataegus-Extrakt (z. B. oligomere Procyanidine), Buchweizen (z. B. Rutin), Sophora japonica (z. B. Rutin), Birkenblätter (z. B. Quercetinglykoside, Hyperosid und Rutin), Holunderblüten (z. B. Rutin), Lindenblüten (z. B. ätherisches Öl mit Quercetin und Farnesol), Johanniskautöl, (z. B. Olivenölauszug), Calendula, Arnika (z. B. ölige Auszüge der Blüten mit ätherischem Öl, polare Auszüge mit Flavonoiden), Melisse (z. B. Flavone, ätherisches Öl); Immunstimulantien: Echinacea purpurea (z. B. alkoholische Auszüge, Frischpflanzensaft, Presssaft), Eleutherokokkus senticosus; Alkaloid: Rauwolfia (z. B. Prajmalln), Immergrün (z. B. Vincamin); weitere Phytopharmaka : Aloe, Rosskastanie (z. B. Aescin), Knoblauch (z. B. Knoblauchöl), Ananas (z. B. Bromelaine), Ginseng (z. B. Ginsenoside), Mariendistelfrüchte (z. B. auf Silymarin standardisierter Extrakt), Mäusedornwurzel (z. B. Ruscogenin), Baldrian (z. B. Valepotriate, Tct. Valerianae), Kava-Kava (z. B. Kavalactone), Hopfenblüten (z. B. Hopfenbitterstoffe), Extr. Passiflorae, Enzian (z. B. ethanol. Extrakt), anthrachinonhaltige Drogenauszüge, z. B. aloinhaltiger Aloe Vera-Saft, Pollenextrakt, Algenextrakte, Süssholzwurzelextrakte, Palmenextrakt, Galphimia (z. B. Urtinktur), Mistel (z. B. wässrig-ethanoi. Auszug), Phytosterole (z. B. beta-Sitosterin), Wollblumen (z. B. wässrig-alkohol. Extrakt), Drosera (z. B. Likörweinextrakt), Sanddornfrüchte (z. B. daraus gewonnener Saft oder Sanddornöl), Eibischwurzel, Primelwurzelextrakt, Frischpflanzenextrakte aus Malve, Beinwell, Efeu, Schachtelhalm, Schafgarbe, Spitzwegerich (z. B. Presssaft), Brennessel, Schölikraut, Petersilie; Pflanzenextrakte aus Norolaena lobata, Tagetes lucida, Teeoma siems, Momordica charantia, und Aloe Vera Extrakte.

Bevorzugte kosmetische Wirkstoffe sind natürliche und synthetische Feuchthaltefaktoren wie z. B. Glycerin, Harnstoff und Ceramide, Hautschutzmittel, Hautaufheller, Vitamine, Antioxidantien, sogenannte Antiagingmittel, antiirritative Mittel, Sonnenschutzmittel, etc.

Weitere bevorzugte kosmetische Wirkstoffe sind natürliche Fette und Öle, d. h. Triglyceride von natürlichen Fettsäuren, beispielsweise aufgrund ihrer rückfettenden und pflegenden Wirkung auf der Haut.

Ein besonders bevorzugter kosmetischer Wirkstoff ist Harnstoff, von dem man annimmt, dass er auch als Lokalanästhetikum wirkt.

Im Unterschied zu den vorstehend beschriebenen im Wesentlichen in der Kosmetik verwendeten Formkörpern handelt es sich bei den therapeutisch verwendeten Formkörpern (Arzneimittel/Medizinprodukte) bevorzugt um solche, die mindestens einen pharmazeutischen bzw. therapeutischen Insbesondere auch dermatologischen Wirkstoff enthalten und die im Sinne des Arzneimittelgesetzes unter anderem dazu bestimmt sind, Krankheiten, Leiden, Körperschäden oder krankhafte Beschwerden zu heilen, zu lindern oder zu verhüten. Auch das Alginat selbst kann jedoch auch als solches als pharmazeutisch/therpeutisch wirksamer Bestandteil angesehen werden.

Die Mittel bzw. Wirkstoffe sind für die äußere Anwendung bestimmt, wobei es sich um hautaktive Wirkstoffe aber auch um transdermale Wirkstoffe handelt kann. Sie schließen beispielsweise ein : Mittel zur Behandlung von Hautkrankheiten, äußerlich anwendbare Analgetika, z. B. Dextropropoxyphen, Pentazocin, Pethidin, Buprenorphin ; Antirheumatika/ Antiphlogistika (NSAR), z. B. Indometacin, Diclofenac, Naproxen, Ketoprofen, Ibuprofen, Flurbiprofen, Salicylsäure undderivate wie Acetylsalicylsäure, Oxicame Steroidhormone, z. B. Betamethason, Dexamethason, Methylprednisolon, Ethinylestradiol, Medroergotamin, Dihydroergotoxin ; Gichtmittel, z. B. Benzbromaron, Allopurinol; Dermatika Externa, einschliesslich antibakterielle Mittel, Antimykotika, antivirale Wirkstoffe, entzündungshemmende Wirkstoffe, juckreizstillenden Wirkstoffe, anästhesierende Wirkstoffe, z. B. Benzocain, Corticoide, Aknemittel, antiparasitäre Wirkstoffe ; äußerlich anwendbare Hormone; Venentherapeutika ; Immunsuppresiva etc. alle für die äusserliche Anwendung.

Bevorzugte therapeutische Mittel sind Analgetika, z. B. Immunsuppressiva, Hormone, Mittel zur Behandlung von Hautkrankheiten, wie der Neurodermitis, der atopischen Dermatitis etc., und Anti-Herpesmittel.

Die erfindungsgemäß hergestellten porösen Formkörper können weiterhin gegebenenfalls einen odermehrere

Hilfsstoffe enthalten.

Hilfsstoffe schließen ein: Füllstoffe, pH-Einstellungsmittel, wie Pufferstoffe, Stabilisatoren, Cosolventien, pharmazeutisch und kosmetisch gebräuchliche oder sonstige Farbstoffe und Pigmente, Konservierungsmittel, Weichmacher, Schmiermittel bzw. Gleitmittel, etc.

Ein besonders bevorzugter Hilfsstoff stellt das Squalan dar. Das Squalan besitzt eine hautberuhigende und hautglättende Wirkung.

Die Erfindung betrifft weiterhin die Verwendung eines Salzes eines mehrwertigen Metallions mit einem mehrzähnigen komplexierenden Anion zur Herstellung von porösen, Alginat-haltigen Formkörpern. Dies meint, dass ein solches Salz als solches bei der Bildung derartiger Formkörper zugesetzt wird also nicht partiell oder vollständig an irgendeiner Stelle der Herstellung derartiger Formkörper gebildet wird.

Durch die vorliegende Erfindung lassen sich poröse Formkörper, enthaltend Alginate mehrwertiger Metallionen, die eine Dicke von mindestens einem Zentimeter, bevorzugt mindestens 2 cm aufweisen und die durch Vernetzen (bzw. Ausfällen) von Alginat-haltigen wässrigen Lösungen mit Salzen mehrwertiger Metallionen und nachfolgender Trocknung der wässrigen Suspension des erhaltenen, vernetzten Alginats erhalten werden, herstellen. Die Dicke des Formkörpers meint dabei den kürzesten Abstand zwischen 2 Punkten in einem solchen Formkörper.

Die Herstellung derartig dicker großformatiger Formkörper mit der gewünschten Nassfestigkeit, insbesondere Nassreißfestigkeit, Schneidfähigkeit etc. war bisher im Stand der Technik nicht möglich.

Diese porösen Formkörper werden bevorzugt nach dem erfindungsgemäßen Verfahren erhalten. Die Verfahren, die die Gefriertrocknung von zerkleinertem unlöslichen Alginaten beinhalteten, führen zu leicht zerfallenden, für die vorliegend anvisierte Verwendung ungeeigneten porösen bzw. schwammartigen Materialien.

Die erfindungsgemäßen porösen Formkörper weisen beim Suspendieren von 1 g des Formkörpers in 100 g Wasser bei 20° C einen pH-Wert der wässrigen Phase von weniger als 7, bevorzugt weniger als 6 auf. Ein solch saurer pH-Wert ist insbesondere bei der kosmetischen Anwendung auf der Haut bevorzugt.

Die erfindungsgemäße poröse Formkörper weist bevorzugt eine Dichte von 0,005 bis 1 g/$cm^3$, bevorzugt von 0,01 bis 0,5 g/$cm^3$ auf (bestimmt nach DIN 53420).

Der erfindungsgemäße poröse Formkörper weist bevorzugt eine Nassreißfestigkeit von mindestens etwa 10 mN/mm Schichtdicke auf (bestimmt nach DIN 53328).

Der erfindungsgemäßen porösen Formkörper bestehen nicht oder im Wesentlichen nicht aus gesponnenen Alginatfasern, wie z. B. Calciumalginat-Fasern.

Die genannten erfindungsgemäßen porösen Formkörper können, wie vorstehend erwähnt zusätzlich mindestens eine weitere Komponente enthalten, ausgewählt aus der Gruppe, die besteht aus: kosmetischen oder medizinischen Wirkstoffen, weiteren natürlichen oder synthetischen Hydrokolloidbildenden Polymeren und kosmetischen oder medizinischen Hilfs-bzw. Zusatzstoffen. Diese können in den erfindungsgemäßen porösen Formkörpern in Mengen von bis zu 0,75 g/g, bevorzugt weniger als 0,5 g/g des Formkörpers enthalten sein.

Die erfindungsgemäßen porösen Formkörper eignen sich hervorragend zur Herstellung von schichtförmigen Formkörpern durch Schneiden der erfindungsgemäßen porösen Formkörper in an sich bekannter Weise. Dies ist beispielsweise mit den durch Gefriertrocknung zerkleinerter unlöslicher Alginate hergestellten schwammartigen Materialien nicht möglich. Durch das Schneiden der erfindungsgemäßen porösen Formkörper werden beispielsweise Schichtdicken von 0,5 bis 20 mm erhalten. Die Erfindung betrifft auch die so erhaltenen schichtförmigen porösen Formkörper. Derartige schichtartige poröse Formkörper eignen sich insbesondere für die äußere Anwendung, wie als kosmetische oder medizinische Auflage, als Wundverbandmaterial, als Wundauflage, als Implantatmaterial, als Zellzuchtmatrix etc.

Die erfindungsgemäßen porösen Formkörper eignen sich weiterhin auch hervorragend zur Herstellung von komprimierten, expandierbaren, schwammartigen Formkörpern, wie sie z. B. in der EP 0901792 der Anmelderin auf Kollagenbasis beschrieben sind. Sie lassen sich in einfacher Weise aus den großformatigen insbesondere nach der Gefriertrocknung erhaltenen porösen Formkörpern durch Ausstanzen und/oder Komprimieren insbesondere auch im industriellen Maßstab herstellen, was nach den Methoden des Standes der Technik bislang nicht ohne weiteres möglich ist.

Derartige Komprimate eignen sich insbesondere für die orale, buccale oder nasale Anwendung, wie zum Beispiel als Sättigungskomprimate, die gegebenenfalls zusätzlich Wirkstoffe, Nahrungsergänzungsstoffe oder Vitamine enthalten können (z. B. DE 19942417).

Aufgrund des schwerlöslichen Charakters der erfindungsgemäßen porösen Formkörper eignen sie sich weiterhin zur Herstellung von wirkstoffbeladenen Formen, aus denen der Wirkstoff kontrolliert, insbesondere verzögert freigegeben wird. Derartige Formen schließen sowohl wirkstoffenthaltende Schwämme, wie Implantate, Vaginal- Suppositorien als auch oral verabreichbare Formen, letztere insbesondere als Komprimate, die im befeuchteten Zustand, wie im Magen auf ein mehrfaches ihres komprimierten Volumens expandieren und den enthaltenden Wirkstoff aus der schwammförmigen Matrix freisetzen (z. B. WO 98/09617) ein.

Offenbart werden weiterhin poröse Formkörper, enthaltend Alginate mehrwertiger Metallionen und Hyaluronsäure und/oder deren Salze und/oder deren Derivate. Diese Formkörper verfügen wie oben bereits dargelegt völlig

überraschend über einen erhöhten Weißgrad, was insbesondere in der kosmetischen aber auch in der medizinischen Anwendung sehr bevorzugt ist. Bezüglich der Zusammensetzung derartiger Hyaluronsäure-haltiger poröser Formkörper kann beispielsweise auf die obigen Ausführungen verwiesen werden. Die Hyaluronsäure-haltigen porösen Formkörper werden bevorzugt nach dem erfindungsgemäßen Verfahren hergestellt.

Offenbart werden weiterhin die Verwendung der erfindungsgemäßen porösen Formkörper bzw. der nach dem erfindungsgemäßen Verfahren erhaltenen Formkörper als kosmetische Mittel. Bevorzugt enthalten die porösen Formkörper für die kosmetische Anwendung Alginate mehrwertiger Metallionen und Hydroxycarbonsäuren, insbesondere Hydroxypolycarbonsäuren wie insbesondere Citronensäure die in der Form des erwähnten mehrzähnigen Komplexbildners bereits bei der Herstellung den erfindungsgemäßen porösen Formkörpern zugesetzt werden kann.

Die Anwendung der erfindungsgemäßen porösen Formkörper in der Kosmetik erfolgt bevorzugt in der Form von kosmetischen Hautauflagen, die befeuchtet auf die Haut aufgetragen und nach einer gewissen Einwirkzeit beispielsweise nach Einziehen der darin enthaltenen Wirkstoffe wieder abgenommen werden. Auch das Alginat selbst entfaltet bereits eine kosmetische Wirkung wie die Hydratation und Glättung der Haut.

Offenbart wird weiterhin die Verwendung der erfindungsgemäßen porösen Formkörper bzw. der nach dem erfindungsgemäßen Verfahren erhaltenen Formkörper zur Herstellung eines medizinischen Produktes. Derartige medizinische Produkte schließen beispielsweise Wundauflagen, transdermale Auflagen, Wundpflaster, Implantate, Substrate zur Züchtung von Zellen, Mittel für die kontrollierte, insbesondere verzögerte Verabreichung von Wirkstoffen in der Form besagter Implantate, aber auch als oral verabreichbare Retardpräparate, oder als sogenannte Sättigungskomprimate, die durch die Expansion des komprimierten porösen Formkörpers im Magen eine Sättigungswirkung entfalten ein. Letztere können auch mit Nahrungsergänzungsmitteln, Vitaminen, Mineralien oder sonstigen Wirkstoffen beaufschlagt werden.

Die erfindungsgemäßen porösen Formkörper bzw. die nach dem erfindungsgemäßen Verfahren erhaltenen Formkörper dienen bevorzugt zur äußeren Anwendung, wie insbesondere als kosmetische oder medizinische Auflage. Daneben sind aber wie erwähnt auch beispielsweise die orale, buccale, vaginale, nasale Anwendung etc. möglich. Die erfindungsgemäß zugänglichen homogenen dicken porösen Alginatformkörper erlauben wie gesagt die Herstellung beliebige dieser Applikationsformen in industriellem Maßstab mit bekannten Methoden, wie Schneiden, Pressen, bzw. Komprimieren und/oder Ausstanzen.

Besonders bevorzugte erfindungsgemäße Formkörper enthalten, bezogen auf die Trockensubstanz also ohne Restfeuchte:

Etwa 6 bis 100 Gew.-% Alginat

0 bis etwa 90 Gew.-% Carboxymethylcellulose, insbesondere deren Natriumsalz,

0 bis etwa 70 Gew.-% Hyaluronsäure bzw. deren Salze oder Derivate,

0 bis etwa 90 Gew. -%natürliche oder synthetische Öle,

0 bis etwa 70 Gew.-% Zitronensäure oder deren Salze,

was Vorzugsgehalten in der zu gefriertrocknenden wässrigen Suspension in Schritt c) von etwa 0,2 bis 3 Gew.-% Alginat

0 bis etwa 3 Gew.-% Carboxymethylcellulose, insbesondere deren Natriumsalz,

0 bis etwa 1 Gew.-% Hyaluronsäure bzw. deren Salze oder Derivate,

0 bis etwa 3 Gew. -% natürliche oder synthetische Öle,

0 bis etwa 1 Gew.-% Zitronensäure oder deren Salze,

entspricht.

Die erfindungsgemäßen porösen Formkörper liegen bevorzugt in Form einer Schicht vor, d. h. Länge und Breite des Formkörpers sind wenigstens 10-mal, bevorzugt wenigstens 20-mal so groß wie die Dicke des Formkörpers. Solche Schichten können auch in Formen geschnitten werden, etwa in Form einer Gesichtsmaske. Die Schichten haben eine Fläche von bevorzugt mindestens etwa 25 $cm^2$, bevorzugter von mindestens etwa 50 $cm^2$, noch bevorzugter von mindestens etwa 100 $cm^2$.

Offenbart werden weiterhin auch Laminate, enthaltend mindestens eine Schicht, wie vorstehend beschrieben, die auf mindestens einer Seite mit mindestens einer weiteren Trägerschicht laminiert ist. Bevorzugt ist die erfindungsgemäße Schicht nur auf einer Seite mit bevorzugt nur einer Trägerschicht laminiert. Die Trägerschicht besteht bevorzugt aus einem Rayonnetz (aus Viskose).

Derartige Laminate finden bevorzugt Verwendung als Wundauflage oder Wundpflaster und besonders bevorzugt als kosmetische Maske.

Offenbart wird auch eine Kombination, enthaltend mindestens einen der erfindungsgemäßen porösen Formkörper sowie mindestens eine wässrige Lösung, die einen oder mehrere Wirkstoffe und/oder Hilfsstoffe enthält, in einer zusammengehörenden, räumlichen Anordnung (Anwendungspaket, Set, Kit-of-Parts etc.). Bei der Wirkstofflösung kann es sich z. B. um Lösungen von leicht-flüchtigen Wirkund/ oder Hilfsstoffen handeln, die aufgrund des Herstellverfahrens durch die Gefriertrocknung nicht in den Formkörper eingebracht werden sollen bzw. können, wie z. B. gewisse Anteile ätherischer Öle, Parfums etc. Weiterhin kann die Lösung temperaturempfindliche pharmazeutische oder kosmetische Wirkstoffe enthalten.

Die Erfindung wird im Hinblick auf die folgenden Beispiele näher erläutert.

BEISPIELE:

BEISPIEL 1

(HERSTELLUNGSWEG 1: Mit mehrzähnigem Liganden komplexiertes Calcium danach Gleichgewichtsverschiebung durch Zugabe von Citronensäure)

Schritt 1:

2500 g RO-Wasser (entsalztes Wasser, Reverse Osmose)
32,5 g Na-Alginat
10,0 g Calciumcitrat
Das Alginatpulver mittels eines Mixers ins RO-Wasser einarbeiten bis eine homogene Mischung entsteht. Danach das Calciumcitrat unterrühren (an dieser Stelle können zweckmäßig auch gegebenenfalls kosmetische und/oder medizinische Wirkstoffe und/oder Öle oder sonstige Stoffe etc. in die Lösung eingearbeitet werden).

Schritt 2:

100 g RO-Wasser
12,5 g Citronensäure
Die Zitronensäure wird unter Rühren in 100 ml RO-Wasser gegeben.

Schritt 3: Die Lösungen aus Schritt 1 und Schritt 2 werden ca. 30 Sekunden lang innig gemischt.

Schritt 4: Die Mischung aus Schritt 3 wird in eine Form gegossen und man lässt für ca. 2 h ausreagieren.

Schritt 5: Der gelierte Formkörper wird schockgefroren und gefriergetrocknet.

Schritt 6: Der gefriergetrocknete, großformatige, poröse bzw. schwammartige, gegebenenfalls mit zusätzlichen Stoffen beladene Formkörper kann wie oben erläutert konfektioniert werden.

BEISPIEL 2

(HERSTELLUNGSWEG 2 (nicht erfindungsgemäß):

Vorlage eines mehrzähnigen Komplexbildners und danach Zugabe schwerlösliches Ca-Salz)

Schritt 1:

2500 g RO-Wasser (entsalztes Wasser, Reverse Osmose)

32,5 g Na-Alginat

12,5 g Citronensäure

Das Alginatpulver mittels eines Mixers ins RO-Wasser einarbeiten bis eine homogene Mischung entsteht. Danach wird die Citronensäure untergerührt (an dieser Stelle können zweckmäßig auch gegebenenfalls kosmetische und/oder medizinische Wirkstoffe und/oder Öle oder sonstige Stoffe etc. in die Lösung eingearbeitet werden).'

Schritt 2:

50 g RO-Wasser
10,0 g Calciumsulfat Das Calciumsulfat wird unter Rühren in 50 ml RO-Wasser gegeben.

Schritt 3: Die Lösungen aus Schritt 1 und Schritt 2 werden ca. 30 Sekunden lang innig gemischt.

Schritt 4: Die Mischung aus Schritt 3 wird in eine Form gegossen und man lässt für ca. 1 h ausreagieren.

Schritt 5: Der gelierte Formkörper wird schockgefroren und gefriergetrocknet.

Schritt 6: Der gefriergetrocknete, großformatige, poröse bzw. schwammartige, gegebenenfalls mit zusätzlichen Stoffen beladene Formkörper kann wie oben erläutert konfektioniert werden.

## Patentansprüche

1. Verfahren zur Herstellung von Alginat-haltigen porösen Formkörpern, dass die Schritte umfasst:

    a) Herstellen einer wässrigen Lösung eines wasserlöslichen Alginates,
    b1) Zugabe eines oder mehrerer Salze eines mehrwertigen Metallions mit einem mehrzähnigen komplexierenden Anion zur wässrigen Lösung des wasserlöslichen Alginates, und Verschiebung des Komplexbildungsgleichgewichtes des mehrwertigen Metallions und des mehrzähnigen komplexierenden Anions unter Erhöhung der verfügbaren Konzentration des mehrwertigen Metallions in der Alginat-Lösung, und dadurch Bildung von Salzen des Alginates mit dem genannten mehrwertigen Metallion,
    c) Giessen der fließfähigen wässrigen Alginat-Zusammensetzung in eine Form,
    d) Gefriertrocknung der wässrigen Alginat-Zusammensetzung unter Bildung des porösen, Alginat-haltigen Formkörpers.

    wobei die Verschiebung des Komplexbildungsgleichgewichtes des mehrwertigen Metallions und des mehrzähnigen komplexierenden Anions in Schritt b1) durch den Zusatz mindestens einer Säure erfolgt.

2. Verfahren nach einem der Ansprüche 1, das zusätzlich vor dem Schritt d) das Zugeben mindestens einer weiteren Komponente, ausgewählt aus der Gruppe, die besteht aus: kosmetischen oder medizinischen Wirkstoffen, weiteren natürlichen oder synthetischen Hydrokolloid-bildenden Polymeren und kosmetischen oder medizinischen Hilfs- bzw. Zusatzstoffen umfasst.

## Claims

1. A process for the manufacture of alginate-containing porous shaped articles, comprising the steps:

    a) preparing an aqueous solution of a water soluble alginate b1) adding one or more salts of a polyvalent metal ion and a multidentate complexing anion to the aqueous solution of the water-soluble alginate, shifting the complex-formation equilibrium of the polyvalent metal ion and the multidentate complexing anion, increasing the available concentration of the polyvalent metal ion in the alginate solution, thereby producing salts of alginate with said polyvalent metal ion,
    c) pouring the flowable aqueous alginate composition into a mold,
    d) drying the aqueous alginate composition to form a porous shaped article, wherein the shifting of the complex-formation equilibrium of the polyvalent metal ion and the multidentate complexing anion in step b1) is achieved by the addition of at least one acid.

2. Process according to claim 1 further comprising, prior to step d), the addition of a further component selected from the group consisting of cosmetic substances, medical substances, further natural of synthetic hydrocolloid-forming polymers and cosmetic or medical adjuvants.

**Revendications**

1. Procédé de fabrication d'objets façonnés poreux contenant de l'alginate, comprenant les étapes consistant à:

   a) préparer une solution aqueuse d'un alginate soluble dans l'eau
   b1) ajouter un ou plusieurs sels d'un ion de métal polyvalent et d'un anion complexant multidentate à la solution aqueuse de l'alginate soluble dans l'eau, décalant l'équilibre de formation du complexe de l'ion métal polyvalent et de l'anion complexant multidentate, augmentant la concentration disponible de l'ion de métal polyvalent dans la solution d'alginate, produisant ainsi des sels d'alginate avec ledit ion de métal polyvalent,
   c) verser la composition aqueuse d'alginate pouvant s'écouler dans un moule,
   d) sécher la composition aqueuse d'alginate pour former un article façonné poreux, dans lequel le décalage de l'équilibre de formation de complexe de l'ion de métal polyvalent et de l'anion complexant multidenté de l'étape b1) est obtenu par l'addition d'au moins un acide.

2. Procédé selon la revendication 1, comprenant en outre, avant l'étape d), l'addition d'un autre composant choisi dans le groupe constitué des substances cosmétiques, des substances médicales, en outre naturelles de polymères synthétiques formant un hydrocolloïde et d'adjuvants cosmétiques ou médicaux.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 5718916 A
- GB 2357765 A
- DE 20219666 U1
- DE 4328329
- WO 0117377 A
- WO 9400512 A
- US 3653383 A
- EP 251905 A
- DE 4328329 C2
- DE 4028622 C2
- EP 0901792 A
- DE 19942417
- WO 9809617 A

### In der Beschreibung aufgeführte Nicht-Patentliteratur

- Römpp Chemie Lexikon. Naturstoffe. Thieme Verlag, 1997
- B. Römpp Chemie Lexikon. Naturstoffe. Thieme Verlag, 1997